# EUROPEAN PATENT SPECIFICATION

(11) **EP 1 677 722 B1**
(45) Date of publication and mention of the grant of the patent: **01.07.2009**
(21) Application number: 04791053.4
(22) Date of filing: 29.10.2004
(51) Int. Cl.: A61F 13/22, A61F 13/20

(54) **TAMPON HAVING LIQUID-RESISTANT BASE**
TAMPON MIT FLÜSSIGKEITSRESISTENTEM BODEN
TAMPON POSSEDANT UNE BASE RESISTANT AUX LIQUIDES

(30) Priority: 31.10.2003 US 700070
(43) Date of publication of application: 12.07.2006
(73) Proprietor: Johnson & Johnson GmbH, 41470 Neuss (DE)
(72) Inventor: AWOLIN, Bernhard, 41546 Kaarst (DE); HIPP, Werner, 24147 Klausdorf (DE)
(74) Representative: Metten, Karl-Heinz
(86) International application number: PCT/EP2004/012296
(87) International publication number: WO 2005/048901

(56) References cited:
- US-A- 3 038 214
- US-A- 5 185 010
- US-A1- 2002 142 693

## Description

### FIELD OF THE INVENTION

The present invention relates to a tampon having an overwrap material disposed on absorbent material, the overwrap material has a liquid-permeable zone and a liquid-resistant zone, and the liquid-resistant zone of the overwrap material forms a fold over an edge of the absorbent material, and to methods for the manufacture of said tampon.

### BACKGROUND OF THE INVENTION

An overwrapped, spirally wound tampon is disclosed in Friese, U.S. Pat. No. 4,816,100. This tampon has a liquid-permeable, thermoplastic strip section bonded by heat-sealing to the outside of the nonwoven web section. The outer end of the strip section, which projects beyond the end of the nonwoven web section, is bonded to the outside of part of the strip section sealed to the nonwoven web section. Both the insertion and withdrawal end of the tampon remain free of the overwrap material. Although heat-sealing this liquid-permeable thermoplastic strip section to the absorbent structure provided a significant advance in the art, significantly reducing the number of absorbent fibers that could slough off of the tampon, additional improvements to tampon leakage continue to be sought.

Another overwrapped, spirally wound tampon is disclosed in Brown, Jr., U.S. Pat. No. 5,185,010. This tampon has an absorbent material wound in a spiral configuration, the outermost winding of the spiral having a liquid permeable overwrap material disposed thereon, a portion of said overwrap being folded over the edge of the spiral which corresponds to the withdrawal end of the tampon and adhered to the inside surface of the outermost winding. While this illustrates an alternative way to bond the cover to the absorbent structure, it fails to address early tampon leakage problems.

Yet another tampon is disclosed in Kraemer, U.S. Ser. No. 10/285,099, filed Oct. 31, 2002. This tampon has an introduction end, with a recovery end, from which a withdrawal string extends. A first region, which extends from the introduction end in the direction of the recovery end, comprises a first material. A second region, located near the recovery end of the tampon, comprises a second material. The first material has a higher absorbency and a higher hydrophilicity than the second material. The diameter of the second region is at least as large as the mean diameter of the first region. During the absorption of fluid, the second region expands at least essentially perpendicularly to the longitudinal axis of the tampon.

In US 2002/0142693 A1 a catamenial absorbent device for insertion into the vaginal cavity is disclosed comprising a lower, fluid-impermeable barrier, an upper, fluid-pervious overwrap and an absorbent material contained therein. The lower portion is an impermeable barrier that prevents fluid from passing from the vaginal canal and is formed on the bottom surface of the absorbent article. This lower portion can be in the form of a cup-like structure. The fluid-pervious overwrap material is sealed to the lower portion and shall prevent users from noticing absorbent material within the absorbent article. With such a structure of a catamenial absorbent device leakage of bodily fluids shall be prevented.

In US 3,038,214 a catamenial tampon is disclosed which can aquire the form of a rectilinear bat or a circular tampon the outer lower end of which is provided with a moisture resistant barrier. This barrier can be a sheet material in the form of a moisture-resistant paper, plastic or fabric which can be affixed to the lower end of a bat from which the tampon is to be formed or compressed by way of stitches.

While there are illustrated developments in providing thermoplastic covers to tampons and attempts to reduce early tampon leakage, a tampon is needed that contains absorbent fibers, that exhibits enhanced ability to reduce early tampon leakage, and that is easily manufactured in modem, high-speed tampon manufacturing equipment.

### SUMMARY OF THE INVENTION

With the present invention a tampon according to features of claim 1 has been found. This tampon offers improved fluid containment within its absorbent structure.

The tampon of the invention is formed by attaching a length of overwrap material having a liquid-permeable zone and a liquid-resistant zone to an absorbent material to form a laminate; folding a portion of the liquid-resistant zone over an edge of the absorbent material; and forming the laminate into a tampon, wherein the folded portion of the liquid-resistant zone of the overwrap material is located at the withdrawal end of the tampon.

In another aspect of the invention, the tampon is formed through a series of steps. A laminate is formed by attaching a plurality of spaced-apart, individual absorbent material web pads to a substantially continuous web of overwrap material having a liquid-permeable zone and a liquid-resistant zone. Each individual absorbent material web pad has a length that is oriented parallel to the substantially continuous length of overwrap material and a width. In addition, a portion of the liquid-resistant zone is folded over an edge of the individual absorbent material web pads corresponding to the withdrawal end of the finished tampon. A construction comprising one individual absorbent material web pad and a section of the overwrap material is separated from the laminate. The construction has a tab formed of an extension of the overwrap material beyond a longitudinal end of the individual absorbent material web pad. A withdrawal string can be looped around an intermediate portion of the construction, generally parallel to the width of the individual absorbent material web pad. Winding the construction about an axis parallel to the width of the individual absorbent material web pad can form a substantially cylindrical tampon blank with the withdrawal string extending from the withdrawal end of it. Attaching the tab to a portion of the overwrap material disposed on the surface of the tampon blank can prevent it from unwinding, and the tampon blank can be formed into a tampon, e.g., by compression.

Other aspects and features of the present invention will become apparent in those ordinarily skilled in the art upon review of the following description of specific embodiments of the invention in conjunction with the accompanying drawings.

### Brief Description of the Drawing

FIG. 1 shows a perspective view of a tampon according to the invention.
FIGS. 2a-f show a diagrammatic view of process steps useful to produce a tampon according to the invention.
FIG. 3 shows cross-section along plane 3-3 in FIG. 2f.
FIG. 4 shows a diagrammatic view of an alternative embodiment (similar to the view of 2a) in which the overwrap includes two different materials
FIG. 5 shows a diagrammatic view of an apparatus for producing a tampon according to the invention.

### Detailed Description of the Preferred Embodiments

As used herein the specification and the claims, the term "spiral" and variants thereof relate to winding around a center or pole and gradually receding from or approaching it.

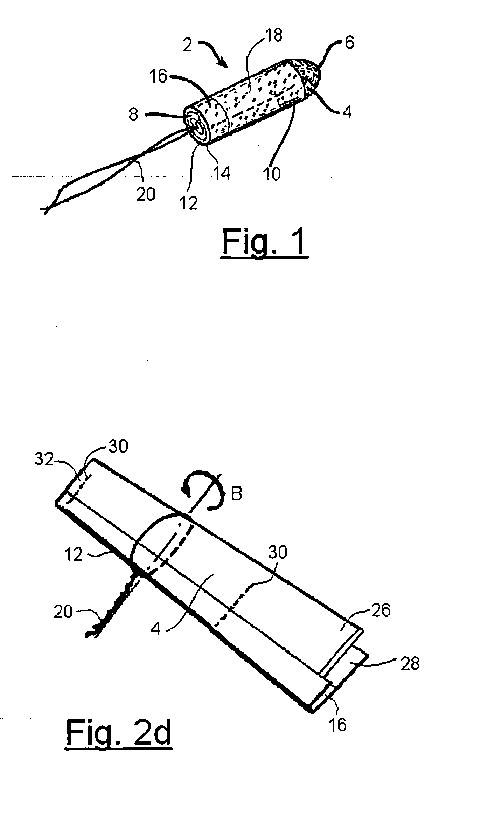

Referring to Fig. 1, there is shown an embodiment of the present invention, a feminine tampon 2. The tampon 2 has an absorbent structure comprising an absorbent material 4, and the tampon 2 has an introduction end 6 and an opposite withdrawal end 8. An overwrap material 10 is disposed on absorbent material 4 as described further, below. The overwrap material 10 forms a fold 12 over an edge 14 of the absorbent material 4, and it has a liquid-permeable zone 16 and a liquid-resistant zone 18. The fold 12 is located in the liquid-resistant zone 18. The tampon 2 also has a withdrawal string 20 extending from the withdrawal end 8.

Absorbent tampons are usually substantially cylindrical masses of compressed absorbent material having a central axis and a radius that defines the outer circumferential surface of the tampon. Tampons are often formed by first obtaining a shaped mass of absorbent material called a tampon blank. This blank can be in the form of a roll of sheet-like material, a segment of a continuous absorbent material, a mass of randomly or substantially uniformly oriented absorbent material, an individually prepared or cast mass of absorbent material, and the like.

The tampon blank is relatively uncompressed and has a relatively low density. The overwrap substantially encloses the tampon blank, and the overwrap encloses a majority of the outer surface of the tampon. The blank may then be compressed to form a product having overall dimensions less than those of the blank prior to use. The compressed tampons may have a generally uniform density throughout the tampon, or they may have regions of differing density as described in the commonly assigned applications to Friese et al., US Pat. No. 6,310,296, and Leutwyler et al., US Pat. No. 5,911,712.

The overwrap can ease the insertion of the tampon into the body cavity and can reduce the possibility of fibers being separated from the tampon. Those of ordinary skill in the art are familiar with materials that are useful in forming overwraps. Overwrap materials may be selected from an outer layer of fibers that are fused together (such as by thermobonding), a nonwoven fabric, an apertured film, or the like.

As indicated above, the overwrap material has at least two zones: a liquid-permeable zone 16 and a liquid-resistant zone 18. This may be achieved by converting a portion of a liquid-permeable material into a liquid-impermeable material, such as by impregnating a liquid-permeable nonwoven web with a liquid-resistant coating. Alternatively, it may be achieved by converting a portion of a liquid-impermeable material into a liquid-permeable material, such as by selectively aperturing a liquid-impermeable plastic film. It may also be achieved by combining two different materials, one liquid-permeable and the other liquid-impermeable.

The materials that may be used in the tampon include fibers, foams, and particles or other discrete materials. The materials may be polymeric or cellulosic. A representative, non-limiting list of useful materials, including fibrous materials, includes, cellulose, polyester, polyvinyl alcohol, polyolefin, polyamine, polyamide, polyacrylonitrile, and the like. A representative, non-limiting list of useful cellulosic fibers includes natural fibers such as cotton, wood pulp, jute, hemp, sphagnum, and the like; and processed materials including cellulose derivatives such as regenerated cellulose (including rayon and lyocell), cellulose nitrate, carboxymethyl cellulose, and the like.

Compressed tampons rebound slightly after moderate mechanical compression toward their original dimensions. Therefore, tampon blanks are generally over-compressed to allow them to rebound slightly to the desired density for use. Over-compression mechanically constricts expansion to prevent the tampon from expanding without added liquid.

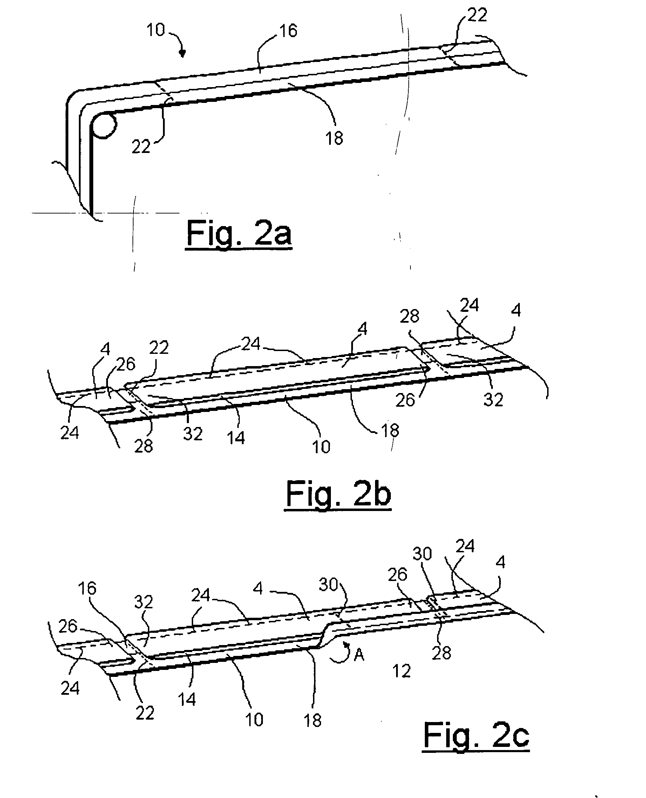

A preferred technique for winding and assembling a tampon of the invention is illustrated in FIGS. 2a-2e.

FIG. 2a shows a substantially continuous length of the overwrap material 10 and the liquid-permeable zone 16 and the liquid-resistant zone 18. Separation lines 22 are also shown, and these lines 22 may be lines of weakness or simply an indication where sections of the overwrap material 10 and absorbent material 4 will later be separated.

As shown in FIG. 2b, sections of absorbent material 4 are attached to overwrap material 10, e.g., through seal 24. Overwrap material 10 extends beyond outer end 26 of absorbent material 4, forming a tab 28. In a preferred embodiment, the overwrap material 10 is cut in a rectangle, and the overwrap material 10 is folded over itself in the area of tab 28, forming a double thickness of material in that area.

In FIG. 2c, overwrap material 10 is folded over edge 18 of absorbent material 4, in the direction indicated by arrow A. A widthwise seal 30 can be formed at one or more places along the construction to retain the overwrap 10 in its folded over position. As shown in FIG. 2c, a widthwise seal 30 may be located near the inner end 32 of the construction and another widthwise seal 30 may be located nearer the outer end 26 of the absorbent material 4.

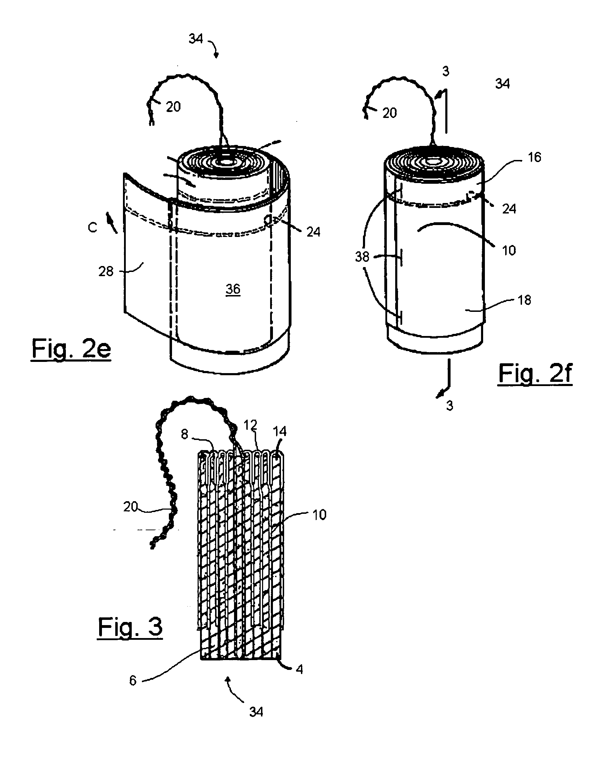

The widthwise seal 30 is also shown in FIG. 2d, and the withdrawal string 20 is looped over an area of the overwrap/absorbent construction near its middle. The construction can then be wound in the direction indicated by arrow B to form the spiral tampon blank 34 shown in FIG. 2e. This may be achieved, generally as disclosed in Friese, U.S. Patent No. 4,816,100. The skilled artisan will recognize that the overwrap of the present invention is the continuous material, while in Friese; it is the absorbent material that is the continuous material. The outermost winding of the spiral (reference number 36 in FIG. 2) is entirely covered by overwrap material 10, and widthwise seal 30 will be covered either by end 26 of absorbent material 4 (preferably) or by tab 28 of overwrap material 10. After the final winding of the absorbent material, tab 28 is wound around the overwrapped surface in the direction indicated by arrow C.

Finally, as shown in FIG. 2f, tab seal 38 attaches tab 28 to the overwrapped surface. In this embodiment, tab seal 38 is a discontinuous (intermittent) seal, to enhance the softness of the sealed area. In other embodiments, tab seal 38 may be continuous. FIG. 3 shows cross-section along plane 3-3 of this structure. In this cross-section, one can see the liquid-impermeable material folded over the edge 14 of the absorbent material 4.

Seals 24, 30, and 38 are preferably all heat seals, and accordingly it is preferred that the overwrap be a heat sealable thermoplastic. It is preferred that the seals be intermittent, particularly the overwrap-to-overwrap seal (seal 38) that will be exposed in the assembled tampon and is thus is preferably soft. However, continuous seals may be used, and the seals may be either smooth or textured, as desired. Appropriate sealing techniques are known in the art.

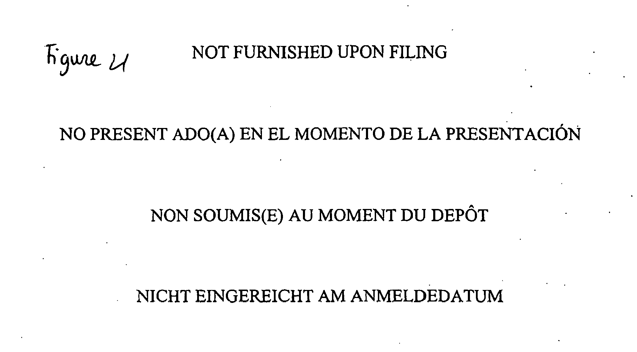

FIG. 4 shows an alternative embodiment in which the overwrap 10 comprises two different materials, one liquid-permeable 16' and the other liquid-impermeable 18'. In this alternative embodiment (similar to FIG. 2a, above), two separate webs are combined, off-set from each other to provide the liquid-permeable zone 16 and the liquid-impermeable zone 18. For example, the liquid-permeable material 16' may be a nonwoven web, an apertured film, or the like, and the liquid-impermeable material 18' may be a nonwoven web, a plastic film, or the like. These two materials may overlap slightly to permit them to be bonded, e.g., by thermobonding, ultrasonic sealing or glueing. The bonding line may be continuous or intermittent.

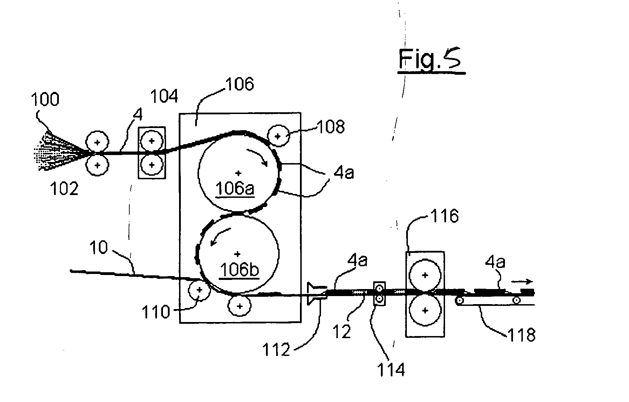

FIG. 5 shows a diagrammatic view of an apparatus for producing a tampon according to the invention. A loose web of fibrous material 100 is fed into a calendar 102 to provide a continuous web of absorbent material 4. The web of absorbent material 4 may be guided into a second calendar, an in-line calendar and feed nip 104, by means of an optional edge guide (not shown). The in-line calendar and feed nip 104 feeds the web of absorbent material 4 to a cut & place unit 106. The cut & place unit 106 may have two rotating vacuum drums 106a and 106b and a cutting roller 108. Preferably, the rotating vacuum drums 106a and 106b rotate faster than the rate at which the absorbent material 4 is provided by the in-line calendar and feed nip 104 to space individual absorbent material pads 4a along the circumference of the rotating vacuum drums 106a and 106b. The first rotating vacuum drum 106a transfers the individual absorbent material pads 4a to the second rotating vacuum drum 106b. As the individual absorbent material pads 4a are carried by the second rotating vacuum drum 106b, they are transferred to a continuous web of overwrap material 10 in the nip between the second rotating vacuum drum 106b and auxiliary roller 110.

After the absorbent material pads 4a are carried out of the cut & place unit 106 by the continuous web of overwrap material 10, a fold-around board 112 folds an edge portion of the liquid-resistant zone of the overwrap material 10 over an edge of the absorbent material pads 4a. The resulting fold 12 is thus located in the liquid-resistant zone.

The overwrap material 10 and the absorbent material 4 can then be secured together in a bonding unit 114. The two elements can be secured through heat and pressure, thermobonding, ultrasonic bonding, and the like. Finally, the construction may pass through a cutting station 116 and onto a conveyor 118. The cutting station 116 may be substantially as described in Friese, U.S. Pat. No. 4,816,100, or it may be a cutting station in which the product is completely severed and delivered to the conveyor 118. Those of ordinary skill in the art will also recognize other suitable technologies for the cutting station 116.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A tampon having an introduction end and an opposite withdrawal end, the tampon comprising a compressed absorbent structure, which structure comprises:
an absorbent material having a length, a width defined from a first edge corresponding to the introduction end of the tampon to a second edge corresponding to the withdrawal end of the tampon, and a thickness; and
an overwrap material disposed on the absorbent material, **characterized in that**
the overwrap material has a length greater than the length of the absorbent material and a width generally corresponding to the width of the absorbent material and comprises a liquid-permeable zone and a liquid-resistant zone, wherein the overwrap material is attached to the absorbent material to form a laminate, wherein the liquid-resistant zone of the overwrap material forms a fold over the second edge of the absorbent material and is disposed at the withdrawal end of the tampon, and wherein said compressed absorbent structure is obtained by spirally winding said absorbent material and said overwrap material disposed on said absorbent material about an axis parallel to the width of the absorbent material.

2. The tampon of claim 1 wherein the absorbent material comprises a fibrous web.

3. The tampon of claim 1 wherein the overwrap material comprises a nonwoven fibrous web.

4. The tampon of claim 1 wherein the overwrap material comprises at least two webs joined together between the fold and the first edge of the absorbent material.

5. The tampon of claim 4 wherein a first web is a nonwoven web that forms the liquid-permeable zone.

6. The tampon of claim 4 wherein a first web is an apertured film that forms the liquid-permeable zone.

7. The tampon of claim 4 wherein a second web is a nonwoven web that forms the liquid-impermeable zone.

8. The tampon of claim 7 wherein the nonwoven web is treated to be liquid-impermeable.

9. The tampon of claim 4 wherein a second web is a polymeric film that forms the liquid-impermeable zone.

10. The tampon of claim 1 wherein the overwrap material comprises a plastic film.

11. The tampon of claim 1 wherein the length of the absorbent material is greater than its width.

12. The tampon of claim 1 wherein the overwrap material has a width, measured parallel to the width of the absorbent material, generally corresponding to the width of the absorbent material.

13. The tampon of claim 12 wherein the width of the overwrap material is not less than the width of the absorbent material.

14. The tampon of claim 12 wherein the liquid-resistant zone comprises a liquid-impermeable structure.

15. A method of forming a tampon having an introduction end and an opposite withdrawal end, the method comprising the steps of:
attaching a length of overwrap material having a liquid-permeable zone and a liquid-resistant zone to an absorbent material to form a laminate, the absorbent material having a length, a width defined from a first edge corresponding to the introduction end of the tampon to a second edge corresponding to the withdrawal end of the tampon; folding a portion of the liquid-resistant zone over the second edge of the absorbent material; and
forming the laminate into a tampon, wherein the folded portion of the liquid-resistant zone of the overwrap material is located at the withdrawal end of the tampon.

16. The method of claim 1 wherein the step of attaching comprises thermobonding.

17. The method of claim 15 wherein the step of attaching comprises adhesive bonding.

18. The method of claim 15 wherein the overwrap material comprises at least two webs joined together between the fold and the first edge of the absorbent material.

19. The method of claim 18 wherein a first web is a nonwoven web that forms the liquid-permeable zone.

20. The method of claim 18 wherein a first web is an apertured film that forms the liquid-permeable zone.

21. The method of claim 18 wherein a second web is a nonwoven web that forms the liquid-impermeable zone.

22. The method of claim 21 wherein the nonwoven web is treated to be liquid-impermeable.

23. The method of claim 18 wherein a second web is a polymeric film that forms the liquid-impermeable zone.

24. A method of forming a tampon having an introduction end and an opposite withdrawal end, the method comprising the steps of:
attaching a plurality of spaced-apart, individual absorbent material web pads to a substantially continuous web of overwrap material having a length, a liquid-permeable zone and a liquid-resistant zone to form a laminate, each individual absorbent material web pad having a length oriented parallel to the substantially continuous length of overwrap material, a width defined from a first edge corresponding to the introduction end of the tampon to a second edge corresponding to the withdrawal end of the tampon;
folding a portion of the liquid-resistant zone over the second edge of the individual absorbent material web pads;
separating a construction comprising one individual absorbent material web pad and a section of the overwrap material from the laminate, the construction having a tab formed of an extension of the overwrap material beyond a longitudinal end of the individual absorbent material web pad;
looping a withdrawal string around an intermediate portion of the construction, generally parallel to the width of the individual absorbent material web pad;
winding the construction about an axis parallel to the width of the individual absorbent material web pad to form a substantially cylindrical tampon blank, with the withdrawal string extending from the withdrawal end of the tampon blank;
attaching the tab to a portion of the overwrap material disposed on the surface of the tampon blank; and
forming the tampon blank into a tampon.

25. The method of claim 24 further comprising providing separation lines substantially perpendicular to the length of the substantially continuous web of overwrap material between the individual absorbent material web pads.

26. The method of claim 25 wherein the step of providing separation lines comprises perforating the overwrap material between the individual absorbent material web pads.

27. The method of claim 25 wherein the step of providing separation lines comprises thinning the overwrap material between the individual absorbent material web pads.

28. The method of either of claims 26 or 27 wherein the step of separating the construction comprises stretching the overwrap material in the vicinity of at least one separation line.

29. The method of claim 24 wherein the step of separating the construction comprises severing the overwrap material between the individual absorbent material web pads.

30. The method of claim 24 wherein the steps of attaching comprise thermobonding.

31. The method of claim 24 wherein the steps of attaching comprise adhesive bonding.

32. The method of claim 24 wherein the step of forming the tampon blank into a tampon comprises compressing the tampon blank.

33. The method of claim 24 wherein the overwrap material comprises at least two webs joined together between the fold and the first edge of the absorbent material.

34. The method of claim 33 wherein a first web is a nonwoven web that forms the liquid-permeable zone.

35. The method of claim 33 wherein a first web is an apertured film that forms the liquid-permeable zone.

36. The method of claim 33 wherein a second web is a nonwoven web that forms the liquid-impermeable zone.

37. The method of claim 36 wherein the nonwoven web is treated to be liquid-impermeable.

38. The method of claim 33 wherein a second web is a polymeric film that forms the liquid-impermeable zone.

## Patentansprüche

1. Tampon mit einem Einführungsende und einem gegenüberliegenden Rückholende, wobei der Tampon eine komprimierte absorbierende Struktur umfasst, und wobei die Struktur umfasst: ein absorbierendes Material mit einer Länge, mit einer Breite, begrenzt von einem ersten Rand aus, welcher dem Einführungsende des Tampons entspricht, bis zu einem zweiten Rand, welcher dem Rückholende des Tampons entspricht, und einer Dicke; und
ein Umhüllungsmaterial, welches auf dem absorbierenden Material angeordnet ist, **dadurch gekennzeichnet, dass** das Umhüllungsmaterial eine Länge aufweist, die größer ist als die Länge des absorbierenden Materials und eine Breite, die im Allgemeinen der Breite des absorbierenden Materials entspricht und eine flüssigkeitsdurchlässige Zone und eine flüssigkeitsresistente Zone umfasst, wobei das Umhüllungsmaterial am absorbierenden Material befestigt ist, um ein Laminat auszubilden, wobei die flüssigkeitsresistente Zone des Umhüllungsmaterials eine Faltung über dem zweiten Rand des absorbierenden Materials ausbildet und am Rückholende des Tampons angeordnet ist, und wobei die komprimierte absorbierende Struktur erhalten wird, indem das absorbierende Material und das Umhüllungsmaterial, welches auf dem absorbierenden Material angeordnet ist, spiralförmig um eine Achse parallel zur Breite des absorbierenden Materials gewickelt werden.

2. Tampon nach Anspruch 1, **dadurch gekennzeichnet, dass** das absorbierende Material ein Fasergewebe umfasst.

3. Tampon nach Anspruch 1, **dadurch gekennzeichnet, dass** das Umhüllungsmaterial ein Faservliesgewebe umfasst.

4. Tampon nach Anspruch 1, **dadurch gekennzeichnet, dass** das Umhüllungsmaterial zumindest zwei Gewebe umfasst, die miteinander zwischen der Faltung und dem ersten Rand des absorbierenden Materials verbunden sind.

5. Tampon nach Anspruch 4, **dadurch gekennzeichnet, dass** ein erstes Gewebe ein Faservliesgewebe ist, welches die flüssigkeitsdurchlässige Zone ausbildet.

6. Tampon nach Anspruch 4, **dadurch gekennzeichnet, dass** ein erstes Gewebe eine mit Öffnungen versehene Schicht ist, welche die flüssigkeitsdurchlässige Zone ausbildet.

7. Tampon nach Anspruch 4, **dadurch gekennzeichnet, dass** ein zweites Gewebe ein Faservliesgewebe ist, welches die flüssigkeitsundurchlässige Zone ausbildet.

8. Tampon nach Anspruch 7, **dadurch gekennzeichnet, dass** das Faservliesgewebe so behandelt ist, dass es flüssigkeitsundurchlässig ist.

9. Tampon nach Anspruch 4, **dadurch gekennzeichnet, dass** ein zweites Gewebe eine polymere Schicht ist, welche die flüssigkeitsundurchlässige Zone ausbildet.

10. Tampon nach Anspruch 1, **dadurch gekennzeichnet, dass** das Umhüllungsmaterial eine Kunststoffschicht umfasst.

11. Tampon nach Anspruch 1, **dadurch gekennzeichnet, dass** die Länge des absorbierenden Materials größer als seine Breite ist.

12. Tampon nach Anspruch 1, **dadurch gekennzeichnet, dass** das Umhüllungsmaterial eine Breite, parallel zur Breite des absorbierenden Materials gemessen, aufweist, welche im Allgemeinen der Breite des absorbierenden Materials entspricht.

13. Tampon nach Anspruch 12, **dadurch gekennzeichnet, dass** die Breite des Umhüllungsmaterials nicht geringer ist als die Breite des absorbierenden Materials.

14. Tampon nach Anspruch 12, **dadurch gekennzeichnet, dass** die flüssigkeitsresistente Zone eine flüssigkeitsundurchlässige Struktur umfasst.

15. Verfahren für das Ausbilden eines Tampons mit einem Einführungsende und einem gegenüberliegenden Rückholende, wobei das Verfahren die folgenden Schritte umfasst:
Befestigen einer Länge von Umhüllungsmaterial, das eine flüssigkeitsdurchlässige Zone und eine flüssigkeitsresistente Zone aufweist, an einem absorbierenden Material, um ein Laminat auszubilden, wobei das absorbierende Material eine Länge, eine Breite aufweist, begrenzt von einem ersten Rand aus, welcher dem Einführungsende des Tampons entspricht, bis zu einem zweiten Rand, welcher dem Rückholende des Tampons entspricht;
Falten eines Abschnitts der flüssigkeitsresistenten Zone über den zweiten Rand des absorbierenden Materials; und
Ausformen des Laminats zu einem Tampon, wobei der gefaltete Abschnitt der flüssigkeitsresistenten Zone des Umhüllungsmaterials sich am Rückholende des Tampons befindet.

16. Verfahren nach Anspruch 15, **dadurch gekennzeichnet, dass** der Schritt des Befestigens Thermobonden umfasst.

17. Verfahren nach Anspruch 15, **dadurch gekennzeichnet, dass** der Schritt des Befestigens Klebebonden umfasst.

18. Verfahren nach Anspruch 15, **dadurch gekennzeichnet, dass** das Umhüllungsmaterial zumindest zwei Gewebe umfasst, die miteinander zwischen der Faltung und dem ersten Rand des absorbierenden Materials verbunden sind.

19. Verfahren nach Anspruch 18, **dadurch gekennzeichnet, dass** ein erstes Gewebe ein Faservliesgewebe ist, welches die flüssigkeitsdurchlässige Zone ausbildet.

20. Verfahren nach Anspruch 18, **dadurch gekennzeichnet, dass** ein erstes Gewebe eine mit Öffnungen versehene Schicht ist, welche die flüssigkeitsdurchlässige Zone ausbildet.

21. Verfahren nach Anspruch 18, **dadurch gekennzeichnet, dass** ein zweites Gewebe ein Faservliesgewebe ist, welches die flüssigkeitsundurchlässige Zone ausbildet.

22. Verfahren nach Anspruch 21, **dadurch gekennzeichnet, dass** das Faservliesgewebe so behandelt ist, dass es flüssigkeitsundurchlässig ist.

23. Verfahren nach Anspruch 18, **dadurch gekennzeichnet, dass** ein zweites Gewebe eine polymere Schicht ist, welche die flüssigkeitsundurchlässige Zone ausbildet.

24. Verfahren der Ausbildung eines Tampons, der ein Einführungsende und ein gegenüberliegendes Rückholende aufweist, wobei das Verfahren die folgenden Schritte umfasst: Befestigen einer Vielzahl von beabstandeten einzelnen Gewebe-Pads aus absorbierendem Material an einer im Wesentlichen durchgängigen Gewebebahn aus Umhüllungsmaterial, mit einer Länge, einer flüssigkeitsdurchlässigen Zone und einer flüssigkeitsresistenten Zone, um ein Laminat auszubilden, wobei jedes einzelne Gewebe-Pad aus absorbierendem Material eine Länge aufweist, die parallel zu der im Wesentlichen durchgängigen Länge des Umhüllungsmaterials ausgerichtet ist, eine Breite, die von einem ersten Rand aus, welcher dem Einführungsende des Tampons entspricht, bis zu einem zweiten Rand, welcher dem Rückholende des Tampons entspricht, begrenzt wird;
Falten eines Abschnitts der flüssigkeitsresistenten Zone über den zweiten Rand der einzelnen Gewebe-Pads aus absorbierendem Material;
Abtrennen einer Konstruktion, welche einen einzelnen Gewebe-Pad aus absorbierendem Material und einen Abschnitt des Umhüllungsmaterials umfasst, vom Schichtpressstoff, wobei die Konstruktion eine Lasche aufweist, ausgebildet aus einer Verlängerung des Umhüllungsmaterials über ein longitudinales Ende des einzelnen Gewebe-Pads aus absorbierendem Material hinaus;
Wickeln eines Rückholbändchens rund um einen Zwischenabschnitt der Konstruktion, im Wesentlichen parallel zur Breite des einzelnen Gewebe-Pads aus absorbierendem Material; Winden der Konstruktion um eine Achse parallel zur Breite des einzelnen Gewebe-Pads aus absorbierendem Material, um einen im Wesentlichen zylindrischen Tamponrohling auszubilden, wobei sich das Rückholbändchen aus dem Rückholende des Tamponrohlings heraus erstreckt;
Befestigen der Lasche an einem Abschnitt des Umhüllungsmaterials, angeordnet auf der Oberfläche des Tamponrohlings; und
Ausbilden des Tamponrohlings zu einem Tampon.

25. Verfahren nach Anspruch 24, welches weiterhin die Bereitstellung von Trennlinien im Wesentlichen senkrecht zur Länge des im Wesentlichen durchgängigen Gewebes aus Umhüllungsmaterial zwischen den einzelnen Gewebe-Pads aus absorbierendem Material umfasst.

26. Verfahren nach Anspruch 25, **dadurch gekennzeichnet, dass** der Schritt der Bereitstellung von Trennlinien das Perforieren des Umhüllungsmaterials zwischen den einzelnen Gewebe-Pads aus absorbierendem Material umfasst.

27. Verfahren nach Anspruch 25, **dadurch gekennzeichnet, dass** der Schritt der Bereitstellung von Trennlinien das Dünnermachen des Umhüllungsmaterials zwischen den einzelnen Gewebe-Pads aus absorbierendem Material umfasst.

28. Verfahren nach einem der Ansprüche 26 oder 27, **dadurch gekennzeichnet, dass** der Schritt des Abtrennens der Konstruktion das Strecken des Umhüllungsmaterials in der Nähe von zumindest einer Trennlinie umfasst.

29. Verfahren nach Anspruch 24, **dadurch gekennzeichnet, dass** der Schritt des Abtrennens der Konstruktion das Durchtrennen des Umhüllungsmaterials zwischen den einzelnen Gewebe-Pads aus absorbierendem Material umfasst.

30. Verfahren nach Anspruch 24, **dadurch gekennzeichnet, dass** die Schritte des Befestigens Thermobonden umfassen.

31. Verfahren nach Anspruch 24, **dadurch gekennzeichnet, dass** die Schritte des Befestigens Klebebonden umfassen.

32. Verfahren nach Anspruch 24, **dadurch gekennzeichnet, dass** der Schritt des Ausformens des Tamponrohlings zu einem Tampon das Komprimieren des Tamponrohlings umfasst.

33. Verfahren nach Anspruch 24, **dadurch gekennzeichnet, dass** das Umhüllungsmaterial zumindest zwei Gewebe umfasst, die miteinander zwischen der Faltung und dem ersten Rand des absorbierenden Materials verbunden sind.

34. Verfahren nach Anspruch 33, **dadurch gekennzeichnet, dass** ein erstes Gewebe ein Faservliesgewebe ist, welches die flüssigkeitsdurchlässige Zone ausbildet.

35. Verfahren nach Anspruch 33, **dadurch gekennzeichnet, dass** ein erstes Gewebe eine mit Öffnungen versehene Schicht ist, welche die flüssigkeitsdurchlässige Zone ausbildet.

36. Verfahren nach Anspruch 33, **dadurch gekennzeichnet, dass** ein zweites Gewebe ein Faservliesgewebe ist, welches die flüssigkeitsundurchlässige Zone ausbildet.

37. Verfahren nach Anspruch 36, **dadurch gekennzeichnet, dass** das Faservliesgewebe so behandelt ist, dass es flüssigkeitsundurchlässig ist.

38. Verfahren nach Anspruch 33, **dadurch gekennzeichnet, dass** ein zweites Gewebe eine polymere Schicht ist, welche die flüssigkeitsundurchlässige Zone ausbildet.

## Revendications

1. Tampon ayant une extrémité d'introduction et une extrémité d'extraction opposée, le tampon comprenant une structure absorbante comprimée, laquelle structure comprend :
un matériau absorbant ayant une longueur, une largeur définie à partir d'un premier bord correspondant à l'extrémité d'introduction du tampon jusqu'à un second bord correspondant à l'extrémité d'extraction du tampon, et une épaisseur ; et
un matériau enveloppant disposé sur le matériau absorbant, **caractérisé en ce que** le matériau enveloppant a une longueur plus grande que la longueur du matériau absorbant et une largeur correspondant de manière générale à la largeur du matériau absorbant et comprend une zone perméable aux liquides et une zone résistant aux liquides, le matériau enveloppant étant fixé sur le matériau absorbant pour former un stratifié, la zone résistant aux liquides du matériau enveloppant formant un pli au-dessus du second bord du matériau absorbant et étant disposée à l'extrémité d'extraction du tampon, et ladite structure absorbante comprimée étant obtenue en enroulant en spirale le matériau absorbant et ledit matériau enveloppant disposé sur ledit matériau absorbant autour d'un axe parallèle à la largeur du matériau absorbant.

2. Tampon selon la revendication 1, dans lequel le matériau absorbant comprend une bande fibreuse.

3. Tampon selon la revendication 1, dans lequel le matériau enveloppant comprend une bande fibreuse non tissée.

4. Tampon selon la revendication 1, dans lequel le matériau enveloppant comprend au moins deux bandes reliées ensemble entre le pli et le premier bord du matériau absorbant.

5. Tampon selon la revendication 4, dans lequel une première bande est une bande non tissée qui forme la zone perméable aux liquides.

6. Tampon selon la revendication 4, dans lequel une première bande est un film perforé qui forme la zone perméable aux liquides.

7. Tampon selon la revendication 4, dans lequel une seconde bande est une bande non tissée qui forme la zone imperméable aux liquides.

8. Tampon selon la revendication 7, dans lequel la bande non tissée est traitée pour être imperméable aux liquides.

9. Tampon selon la revendication 4, dans lequel une seconde bande est un film de polymère qui forme la zone imperméable aux liquides.

10. Tampon selon la revendication 1, dans lequel le matériau enveloppant comprend un film plastique.

11. Tampon selon la revendication 1, dans lequel la longueur du matériau absorbant est plus grande que sa largeur.

12. Tampon selon la revendication 1, dans lequel le matériau enveloppant a une largeur, mesurée parallèlement à la largeur du matériau absorbant, correspondant généralement à la largeur du matériau absorbant.

13. Tampon selon la revendication 12, dans lequel la largeur du matériau enveloppant n'est pas plus petite que la largeur du matériau absorbant.

14. Tampon selon la revendication 12, dans lequel la zone résistant aux liquides comprend une structure imperméable aux liquides.

15. Procédé de formation d'un tampon ayant une extrémité d'introduction et une extrémité d'extraction opposée, le procédé comprenant les étapes consistant à :
fixer une longueur de matériau enveloppant ayant une zone perméable aux liquides et une zone résistant aux liquides sur un matériau absorbant pour former un stratifié, le matériau absorbant ayant une longueur, une largeur définie à partir d'un premier bord correspondant à l'extrémité d'introduction du tampon jusqu'à un second bord correspondant à l'extrémité d'extraction du tampon ;
plier une partie de la zone résistant aux liquides sur le second bord du matériau absorbant ; et
former le stratifié sous forme d'un tampon, la partie pliée de la zone résistant aux liquides du matériau enveloppant étant située à l'extrémité d'extraction du tampon.

16. Procédé selon la revendication 15, dans lequel l'étape de fixation comprend une thermofixation.

17. Procédé selon la revendication 15, dans lequel l'étape de fixation comprend une fixation par adhésif.

18. Procédé selon la revendication 15, dans lequel le matériau enveloppant comprend au moins deux bandes reliées ensemble entre le pli et le premier bord du matériau absorbant.

19. Procédé selon la revendication 18, dans lequel une première bande est une bande non tissée qui forme la zone perméable aux liquides.

20. Procédé selon la revendication 18, dans lequel une première bande est un film perforé qui forme la zone perméable aux liquides.

21. Procédé selon la revendication 18, dans lequel une seconde bande est une bande non tissée qui forme la zone imperméable aux liquides.

22. Procédé selon leur négation 21, dans lequel la bande non tissée est traitée pour être imperméable aux liquides.

23. Procédé selon la revendication 18, dans lequel une seconde bande est un film de polymère qui forme la zone imperméable aux liquides.

24. Procédé de formation d'un tampon ayant une extrémité d'introduction et une extrémité d'extraction opposée, le procédé comprenant les étapes consistant à:
fixer une pluralité de segments individuels de matériau absorbant, espacés, sur une bande sensiblement continue de matériau enveloppant ayant une longueur, une zone perméable aux liquides et une zone résistant aux liquides, pour former un stratifié, chaque segment individuel de bande de matériau absorbant ayant une longueur orientée parallèlement à la longueur sensiblement continue du matériau enveloppant, une largeur définie depuis un premier bord correspondant à l'extrémité d'introduction du tampon jusqu'à un second bord correspondant à l'extrémité d'extraction du tampon ;
plier une partie de la zone résistant aux liquides sur le second bord des segments individuels de bande de matériau absorbant ;
séparer une construction comprenant un segment individuel de bande de matériau absorbant et un tronçon du matériau enveloppant à partir du stratifié, la construction ayant une patte formée d'un prolongement du matériau enveloppant au-delà d'une extrémité longitudinale du segment individuel de bande de matériau absorbant ;
mettre en boucle un cordon d'extraction autour d'une partie intermédiaire de la construction, de manière générale parallèlement à la largeur du segment individuel de bande de matériau absorbant ;
enrouler la construction autour d'un axe parallèle à la largeur du segment individuel de bande de matériau absorbant pour former une ébauche de tampon sensiblement cylindrique, le cordon d'extraction s'étendant à partir de l'extrémité d'extraction de l'ébauche de tampon ;
relier la patte à une partie du matériau enveloppant disposé sur la surface de l'ébauche de tampon ; et
former l'ébauche de tampon en tampon.

25. Procédé selon la revendication 24, comprenant de plus la fourniture de lignes de séparation sensiblement perpendiculaires à la longueur de la bande sensiblement continue de matériau enveloppant entre les segments individuels de bande de matériau absorbant.

26. Procédé selon la revendication 25, dans lequel l'étape de fourniture de lignes de séparation comprend la perforation du matériau enveloppant entre les segments individuels de bande de matériau absorbant.

27. Procédé selon la revendication 25, dans lequel l'étape de fourniture de lignes de séparation comprend l'amincissement du matériau enveloppant entre les segments individuels de bande de matériau absorbant.

28. Procédé selon la revendication 26 ou 27, dans lequel l'étape de séparation de la construction comprend l'étirement du matériau enveloppant au voisinage d'au moins une ligne de séparation.

29. Procédé selon la revendication 24, dans lequel l'étape de séparation de la construction comprend la découpe du matériau enveloppant entre les segments individuels de bande de matériau absorbant.

30. Procédé selon la revendication 24, dans lequel les étapes de fixation comprennent une thermofixation.

31. Procédé selon la revendication 24, dans lequel les étapes de fixation comprennent une fixation par adhésif.

32. Procédé selon la revendication 24, dans lequel l'étape de formation de l'ébauche de tampon en tampon comprend la compression de l'ébauche de tampon.

33. Procédé selon la revendication 24, dans lequel le matériau enveloppant comprend au moins deux bandes reliées l'une à l'autre entre le pli et le premier bord du matériau absorbant.

34. Procédé selon la revendication 33, dans lequel une première bande est une bande non tissée qui forme la zone perméable aux liquides.

35. Procédé selon la revendication 33, dans lequel une première bande est un film perforé qui forme la zone perméable aux liquides.

36. Procédé selon la revendication 33, dans lequel une seconde bande est une bande non tissée qui forme la zone imperméable aux liquides.

37. Procédé selon la revendication 36, dans lequel la bande non tissée est traitée pour être imperméable aux liquides.

38. Procédé selon la revendication 33, dans lequel une seconde bande est un film de polymère qui forme la zone imperméable aux liquides.
